# EUROPÄISCHE PATENTSCHRIFT

(11) **EP 1 715 341 B1**
(45) Veröffentlichungstag und Bekanntmachung des Hinweises auf die Patenterteilung: **27.02.2008**
(21) Anmeldenummer: 05007284.2
(22) Anmeldetag: 04.04.2005
(51) Int. Cl.: G01N 33/543, G01N 33/558

(54) **Verfahren und Vorrichtung zum Bestimmen der Konzentrationen von mindestens zwei Liganden**
Method and apparatus for determining the concentrations of at least 2 ligands.
Procédé et dispositif pour déterminer les concentrations d'au moins deux ligands.

(43) Veröffentlichungstag der Anmeldung: 25.10.2006
(73) Patentinhaber: Micronas Holding GmbH, 79108 Freiburg i.Br. (DE)
(72) Erfinder: Klapproth, Holger, Dr., 79108 Freiburg (DE)
(74) Vertreter: Huwer, Andreas

(56) Entgegenhaltungen:
- WO-A-92/18866
- WO-A-95/18377
- WO-A-95/22766

## Beschreibung

Die Erfindung betrifft ein Verfahren nach dem Oberbegriff von Anspruch 1 und eine Vorrichtung nach dem Oberbegriff von Anspruch 7.

Eine derartige Vorrichtung ist aus US 6 197 503 B1 bekannt. Sie weist eine Durchfluss-Messkammer auf, die an ihrer Unterseite durch ein etwa plattenförmiges Substrat begrenzt ist, auf dem mehrere Teststellen angeordnet sind, an denen Rezeptoren immobilisiert sind. Die Rezeptoren sind jeweils für einen bestimmten, in einer zu analysierenden Probe enthaltenden Liganden bindungsspezifisch. An der Rückseite des Substrats ist ein Halbleiterchip vorgesehen, der an den einzelnen Teststellen jeweils einen optischen Sensor aufweist. Zur Anregung der Emission von Lumineszenzstrahlung in Abhängigkeit von der Bindung der Liganden an die Rezeptoren weist die Vorrichtung eine optische Strahlungsquelle auf, in deren Abstrahlbereich die Rezeptoren angeordnet sind. Zum Messen der Konzentration der Liganden wird die Probe durch eine Einlassöffnung hindurch derart in die Messkammer eingebracht, dass die in der Probe enthaltenen Liganden an die Rezeptoren binden können. Nach Ablauf einer vorbestimmten Einwirkungsdauer werden nicht an einen Rezeptor gebundenen Bestandteile der Probe aus der Messkammer entfernt. Die Einwirkungsdauer wird so gewählt, dass nach dem Entfernen der ungebundnen Bestandteile nur ein Teil der an den einzelnen Teststellen jeweils vorhandenen Bindungsstellen eines Rezeptors an den für den betreffenden Rezeptor bindungsspezifischen Liganden gebunden ist. Dabei ist das Verhältnis der gebundenen Bindungsstellen zu den freien Bindungsstellen von der Konzentration des betreffenden Liganden in der Probe abhängig.

In einem weiteren Verfahrensschritt wird eine Lösung in die Messkammer eingebracht, die Nachweisantikörper enthält, die mit einem Marker markiert sind. Die Nachweisantikörper sind für die Liganden bindungsspezifisch und binden an diese. Danach wird die Lösung aus der Messkammer entfernt, um die Teststellen mit Hilfe der Strahlungsquelle mit der Anregungsstrahlung zu bestrahlen. Die über die Liganden indirekt an die Rezeptoren gebundenen Nachweisantikörper werden durch die Anregungsstrahlung zur Aussendung einer Lumineszenzstrahlung angeregt, deren Wellenlänge sich von der Wellenlänge der Anregungsstrahlung unterscheidet. Mit Hilfe der optischen Sensoren, die für die Lumineszenzstrahlung empfindlich und für die Anregungsstrahlung unempfindlich sind, wird für jede Teststelle jeweils ein von der Lumineszenzstrahlung und somit der Konzentration der Liganden in der Probe abhängiges Messsignal erzeugt.

Die Vorrichtung hat den Nachteil, dass sie nur in einem begrenzten Konzentrationsbereich eine gleichzeitige Bestimmung der einzelnen Konzentrationswerte der unterschiedlichen, in der Probe enthaltenen Liganden ermöglicht. Bei bestimmten Proben, wie zum Beispiel Blutproben, kann es jedoch vorkommen, dass die in der Probe enthaltenen Liganden Konzentrationsunterschiede von bis zu sechs Dekaden aufweisen. Damit bei einer solchen Probe sowohl für den Liganden mit der geringsten Konzentration als auch für den Liganden mit der höchsten Konzentration jeweils ein Konzentrationswert bestimmt werden kann, ohne dass es an einer der Teststellen alle vorhandenen Rezeptor-Bindungsstellen an einen Liganden binden und somit der entsprechende Messwert in die Begrenzung gerät, werden mindestens zwei Versuche durchgeführt. Bei einem ersten, zur Bestimmung der Konzentration des den höchsten Konzentrationswert aufweisenden Liganden vorgesehenen Versuch wird eine geringe Einwirkungsdauer und bei einem zweiten, zur Bestimmung der Konzentration des den niedrigsten Konzentrationswert aufweisenden Liganden vorgesehenen Versuch, wird eine wesentlich größere Einwirkungsdauer gewählt als bei dem ersten Versuch. Nachteilig ist dabei, dass für jeden Versuch jeweils ein neuer Halbleiterchip benötigt wird, weshalb die Messung noch relativ teuer und arbeitsaufwändig ist. Ungünstig ist außerdem, dass eine entsprechend große Probenmenge benötigt wird. Bei der Untersuchung von Blutproben, die mit Hilfe eines Stechwerkzeugs aus dem Finger eines Patienten entnommen wird, kann dies bedeuten, dass an mindestens zwei Stellen Blut aus dem Finger entnommen werden muss, was besonders bei Kindern problematisch ist.

Aus WO 92/18866 A1 ist ferner ein Verfahren zum Bestimmen der Konzentrationen von mindestens zwei Liganden in einer zu analysierenden Probe bekannt, bei dem jeder Ligand zum Konkurrieren mit einem einzigen Kompetitor fähig ist. Der Kompetitor hat zwei Bindungsdomönen, von denen eine erste Bindungsdomäne zum Binden eines ersten Rezeptors und eine zweite Bindungsdomäne zum Binden eines zweiten Rezeptors fähig sind. Bei dem Verfahren werden zunächst auf einem Festphasen-Substrat an einer ersten Teststelle der erste Rezeptor und an einer zweiten Teststelle der zweite Rezeptor immobilisiert. Dann werden die Probe, der Kompetitor und die aus dem Substrat sowie den ersten und zweiten Rezeptoren bestehende Festphase derart zusammengebracht, dass der erste Ligand und/oder der Kompetitor an den ersten Rezeptor und der zweite Ligand und/oder der Kompetitor an den zweiten Rezeptor binden können. Danach werden Bestandteile des Gemischs, die nicht an einen immobilisierten Rezeptor gebunden sind, von dem Substrat entfernt. Dann werden ein von der Menge des an den ersten Rezeptor gebundenen Kompetitors abhängiges erstes Messsignal und ein von der Menge des an den zweiten Rezeptor gebundenen Kompetitors abhängiges zweites Messsignal erzeugt. Das erste Messsignal wird mit der Menge des ersten Liganden und das zweite Messsignal mit der Menge des zweiten Liganden in der Probe in Beziehung gesetzt. Auch dieses Verfahren hat den Nachteil, dass es nur in einem begrenzten Konzentrationsbereich eine gleichzeitige Bestimmung der einzelnen Konzentrationswerte der unterschiedlichen, in der Probe enthaltenen Liganden ermöglicht.

Es besteht deshalb die Aufgabe, eine Vorrichtung und ein Verfahren der eingangs genannten Art zu schaffen, die eine einfache, schnelle und kostengünstige Messung der Konzentration mehrerer Liganden ermöglichen, von denen vermutet wird, dass sie in einer zu untersuchenden Probe enthalten sind.

Diese Aufgabe wird bezüglich des Verfahrens dadurch gelöst, dass ein für den zweiten Rezeptor bindungsspezifischer Kompetitor derart mit der Probe vermischt wird, dass der Kompetitor in einer vorgegebenen Konzentration in dem so erhaltenen Gemisch vorliegt, dass das Gemisch derart mit dem Substrat in Kontakt gebracht wird, dass der erste Ligand an den ersten Rezeptor und der zweite Ligand und/oder der Kompetitor an den zweiten Rezeptor binden können, dass danach Bestandteile des Gemischs, die nicht an einen immobilisierten Rezeptor gebunden sind, von dem Substrat entfernt werden, dass dann ein von der Konzentration der an den ersten Rezeptor gebundenen ersten Liganden abhängiges erstes Messsignal und ein von der Konzentration des an den zweiten Rezeptor gebundenen Kompetitors abhängiges zweites Messsignal erzeugt werden, und wobei mit Hilfe des ersten Messsignals die Konzentration des ersten Liganden in der Probe und mit Hilfe des zweiten Messsignals und der bekannten Konzentration des Kompetitors in dem Gemisch die Konzentration des zweiten Liganden in der Probe bestimmt werden. Die Liganden und/oder Rezeptoren können Nukleinsäuren oder Derivate davon (DNA, RNA PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen.

In vorteilhafter Weise ist es durch die Kombination des kompetitiven Assays mit einem nicht kompetitiven Assay in derselben Messkammer möglich, bei Proben, die mehrere, in ihrer Konzentration stark voneinander abweichende Liganden enthalten, die Konzentrationen der einzelnen Liganden mit nur einem einzigen Versuch zu bestimmen. Die Konzentration des Kompetitors in dem aus der Probe und dem Kompetitor bestehenden Gemisch und die Einwirkungsdauer, während der das Gemisch mit den Rezeptoren in Kontakt steht, werden dabei so auf die für die einzelnen Liganden zu überprüfenden Konzentrationsbereiche abgestimmt, dass nach dem Entfernen der nicht an einen Rezeptor gebundnen Bestandteile des Gemischs an allen Teststellen nur ein Teil der Bindungsstellen Rezeptoren an einen Liganden bindet, wenn dieser in dem zu untersuchenden Konzentrationsbereich in der Probe enthalten ist. Die Konzentration des Kompetitors in dem Gemisch wird vorzugsweise so gewählt, dass sie einem Grenzwert der Konzentration des zweiten Liganden entspricht, aus dem sich eine Aussage, wie z.B. gut/schlecht" oder "krank/gesund", ableiten lässt. Mit dem erfindungsgemäßen Verfahren können in einer Flusszelle oder dergleichen Messkammer nebeneinander Liganden-Konzentrationen in einem Bereich von g/l bis µg/l nachgewiesen werden.

Bei einer vorteilhaften Ausführungsform der Erfindung wird ein für den ersten Liganden bindungsspezifischer, mit einem ersten Marker markierter Nachweisantikörper mit der ersten Teststelle in Kontakt gebracht, wobei danach nicht an einen immobilisierten Rezeptor gebundene Marker von dem Substrat entfernt werden, und wobei danach das erste Messsignal in Abhängigkeit von der Konzentration des ersten Markers erzeugt wird. Die Konzentration des ersten Liganden kann also mit Hilfe eines Sandwich-Elisa gemessen werden.

Bei einer bevorzugten Ausgestaltung der Erfindung wird der Kompetitor mit einem zweiten Marker markiert, wobei nach dem Entfernen der nicht an einen immobilisierten Rezeptor gebunden Bestandteile des Gemischs von dem Substrat das zweite Messsignal in Abhängigkeit von der Konzentration des an den zweiten Rezeptor gebundenen zweiten Markers erzeugt wird. Dabei kann der zweite Marker mit dem ersten Marker übereinstimmen.

Vorteilhaft ist, wenn der erste Marker und/oder zweite Marker ein Enzym ist (sind), wenn das Enzym während der Erfassung der Messsignale mit mindestens zwei Chemikalien in Kontakt gebracht wird, zwischen denen bei Anwesenheit des Enzyms eine chemische Redoxreaktion auftritt, und wenn das erste Messsignal und/oder das zweite Messsignal durch Messen eines Redoxpotentials erzeugt wird (werden). Dabei kann das Redoxpotential mit Hilfe eines ISFET gemessen werden.

Bei einer zweckmäßigen Ausgestaltung der Erfindung wird (werde n) der erste Marker und/oder zweite Marker während der Erfassung der Messsignale mit einer Anregungsstrahlung bestrahlt, welche den (die) Marker zur Abgabe einer Lumineszenzstrahlung anregt, wobei das erste Messsignal und/oder das zweite Messsignal durch Messen der Lumineszenzstrahlung des betreffenden Markers erzeugt wird (werden). Dabei kann die Anregungsstrahlung mit Hilfe einer Lichtquelle, beispielsweise einer Leuchtdiode und/oder einer Xenon-Lampe erzeugt werden. Für den ersten und zweiten Marker wird vorzugsweise dieselbe Anregungsstrahlung verwendet. Es ist aber auch denkbar, dass der erste und zweite Marker unterschiedliche Farbstoffe, wie z.B. Cy3 und Cy5 sind, und dass diese Farbstoffe mit unterschiedlichen Wellenlängen angeregt werden.

Vorteilhaft ist, wenn die erste Teststelle und/oder zweite Testelle während der Erfassung der Messsignale mit einem Chemilumineszenzsubstrat in Kontakt gebracht wird (werden), in dem in Abhängigkeit von der Bindung des ersten Liganden an den ersten Rezeptor und/oder in Abhängigkeit von der Bindung des Kompetitors an den zweiten Rezeptor zur Abgabe einer Lumineszenzstrahlung angeregt wird, wobei das erste Messsignal und/oder das zweite Messsignal durch Messen der Lumineszenzstrahlung an der betreffenden Teststelle erzeugt wird (werden). Die Lumineszenzstrahlung wird dabei ohne eine Anregungsstrahlung auf chemischem Wege erzeugt.

Die vorstehend genannte Aufgabe wird bezüglich der Vorrichtung dadurch gelöst, dass sie eine Mischeinrichtung aufweist, die zum Vermischen der Probe mit einem für den zweiten Rezeptor bindungsspezifischen Kompetitor mit einer Zuführöffnung für die Probe und einem den Kompetitor enthaltenden Aufnahmeraum verbunden ist, dass die Mischeinrichtung eine Abgabeöffnung für das aus der Probe und dem Kompetitor gebildete Gemisch aufweist, die mit der Einlassöffnung der Messkammer verbunden ist, dass die Mischeinrichtung derart ausgestaltet ist, dass der Kompetitor in einer vorgegebenen Konzentration in dem Gemisch vorliegt, dass der zweite Sensor zum Erfassen eines von der Konzentration des an den zweiten Rezeptor gebundenen Kompetitors abhängigen zweiten Messsignals ausgebildet und mit einer Auswerteeinrichtung verbunden ist, die zum Bestimmen der Konzentration des zweiten Liganden in der Probe aus dem zweiten Messsignal und der Konzentration des Kompetitors in dem Gemisch ausgebildet ist.

Die Probe wird also vor dem Eintritt in die Messkammer mit Hilfe der Mischeinrichtung mit einem für den zweiten Rezeptor bindungsspezifischen Kompetitor vermischt, so dass in der Mischkammer für den zweiten Ligand ein kompetitives Assays und gleichzeitig für den ersten Ligand ein nicht kompetitives Assay durchgeführt werden kann. Wie bereits bei dem Verfahren erläutert wurde, ermöglicht die Kombination dieser Essays bei einer Probe, die mehrere, in ihrer Konzentration stark voneinander abweichende Liganden enthält, die Konzentrationen der einzelnen Liganden mit nur einem einzigen Versuch zu bestimmen.

Vorteilhaft ist, wenn der Kompetitor in gelförmiger, teigiger oder fester Form in dem Aufnahmeraum stabilisiert ist, vorzugsweise derart, dass er an einer Begrenzungswand des Aufnahmeraums anhaftet, und wenn der Aufnahmeraum zum Lösen des Kompetitors in der Probe als Durchflussmischkammer ausgebildet ist, über welche die Zuführöffnung für die Probe mit der Einlassöffnung der Messkammer verbunden ist. Die Vorrichtung ist dadurch besonders gut handhabbar. Die Stabilisierung des Kompetitor umfasst vorzugsweise mindestens ein nicht-reduzierendes Disaccharid und mindestens ein Protein oder Polypeptid der LEA-Klasse. Das nichtreduzierende Disaccharid kann aus einer Gruppe ausgewählt sein, die aus Trehaose (D-Glucopyranosyl-D-glucopyranose), Sucrose (β-D-Fructofüranosyl-α-D-glucopyranosid) sowie Derivaten davon ausgewählt sein kann. Eine derartige Stabilisierung ist in WO 2004/004455 A2 beschrieben. Der in stabilisierter Form vorliegende Kompetitor ist über längere Zeit haltbar.

Bei einer bevorzugten Ausgestaltung der Erfindung weist die Durchflussmischkammer eine Mischerstruktur auf, die derart ausgestaltet ist, dass das Gemisch beim Durchfluss durch die Durchftussmischkammer vorzugsweise in abwechselnd zueinander entgegengesetzt verlaufende Richtungen abgelenkt wird, und dass die Mischerstruktur zwischen dem in gelförmiger, teigiger oder fester Form vorliegenden Kompetitor und der Einlassöffnung der Messkammer angeordnet ist. Dabei kann der Mischer ein so genannter Möbiusmischer sein, der mit Methoden der Mikrosystemtechnik mit sehr kompakten Abmessungen hergestellt werden kann.

Bei einer zweckmäßigen Ausführungsform der Erfindung sind die Sensoren optische Sensoren, wobei der erste Rezeptor zur Detektion einer in Abhängigkeit von der Bindung des ersten Liganden an den ersten Rezeptor erzeugten ersten Lumineszenzstrahlung vorzugsweise direkt auf dem ersten Sensor und/oder der zweite Rezeptor zur Detektion einer in Abhängigkeit von der Bindung des Kompetitors an den zweiten Rezeptor erzeugten zweiten Lumineszenzstrahlung vorzugsweise direkt auf dem zweiten Sensor angeordnet ist (sind). Die an den Rezeptoren erzeugte Lumineszenzstrahlung kann dann direkt ohne den Umweg über eine Sammellinse in den (die) Sensor(en) eingekoppelt werden.

Vorteilhaft ist, wenn der Kompetitor mit einem Marker markiert ist, der durch Bestrahlung mit einer Anregungsstrahlung zur Abgabe der Lumineszenzstrahlung anregbar ist, wenn die Vorrichtung zum Bestrahlen der zweiten Teststelle mit der Anregungsstrahlung mindestens eine Strahlungsquelle aufweist, und wenn der zweite Sensor für die Lumineszenzstrahlung empfindlich und für die Anregungsstrahlung unempfindlich ist. Dabei kann die Strahlungsquelle auch außerhalb der Messkammer angeordnet sein, wenn diese mindestens einen Wandungsbereich hat, der für die Anregungsstrahlung durchlässig ist.

Die Vorrichtung kann Bestandteil eines Kits nach einem der Ansprüche 12 bis 16 sein, der zusätzlich zu der der Vorrichtung
- einen mit einem Enzym oder dergleichen Marker markierten, für den ersten Liganden bindungsspezifischen Nachweisantikörper,
- mindestens zwei Chemikalien, zwischen denen bei einem Kontakt mit dem an dem Nachweisantikörper und/oder dem Kompetitor und/oder dem Nachweisantikörper angeordneten Enzym eine chemische Redoxreaktion auftritt, und/oder
- ein Chemilumineszenzsubstrat, in dem bei einem Kontakt mit dem an dem Nachweisantikörper und/oder dem Kompetitor angeordneten Enzym eine chemische Reaktion ausgelöst wird, bei der eine Lumineszenzstrahlung freigesetzt wird, und/oder
- eine Strahlungsquelle, die zur Aussendung der Lumineszenzstrahlung auf die erste Teststelle angeordnet ist,
aufweisen kann. Der Marker, das Chemilumineszenzsubstrat und/oder die Chemikalien können mit einer geeigneten Zuführeinrichtung, wie z.B. einer Mikropumpe oder einer Pipette, der Messkammer zugeführt werden.

Nachfolgend ist ein Ausführungsbeispiel der Erfindung anhand der Zeichnung näher erläutert. Es zeigen:
- Fig. 1: einen Längsschnitt durch eine Vorrichtung zum Bestimmen der Konzentrationen von in einer zu analysierenden Probe enthaltenen Liganden,
- Fig. 2: eine schematische Darstellung eines Sandwich ELISA,
- Fig. 3: eine schematische Darstellung eines kompetitiven ELISA,
- Fig. 4: eine graphische Darstellung von bei zwei Sandwich ELISA ermittelten Messwerten für die Konzentration eines mit Cy5 markierten Liganden, wobei auf der Abszisse die Zeit und auf der Ordinate die Messwertamplitude aufgetragen sind und wobei die Konzentration als Parameter angegeben ist, und
- Fig. 5: eine graphische Darstellung von bei drei direkten ELISA ermittelten Messwerten für die Konzentration eines mit Cy5 markierten Liganden, wobei auf der Abszisse die Zeit und auf der Ordinate die Messwertamplitude aufgetragen sind und wobei die Konzentration als Parameter angegeben ist.

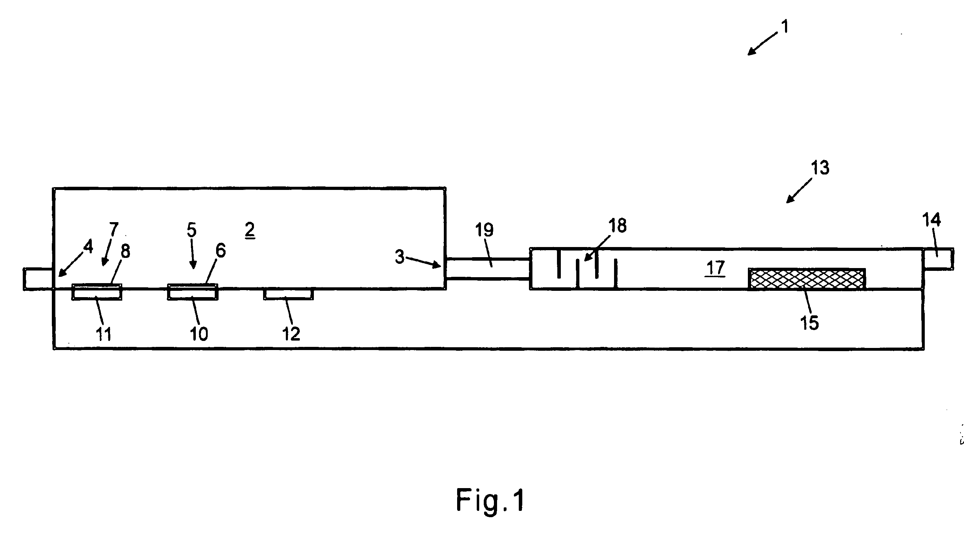

Eine in Fig. 1 im Ganzen mit bezeichnete Vorrichtung zum Bestimmen der Konzentrationen von mindestens zwei Liganden in einer zu analysierenden Probe weist eine als Flusszelle ausgebildete Messkammer 2 auf, die eine Einlassöffnung 3 und eine Auslassöffnung 4 hat. Eine Wandung der Messkammer 2 ist durch ein Halbleiter-Substrat gebildet, auf dem an einer ersten Teststelle 5 ein erster Rezeptor 6 und an einer seitlich davon beabstandeten zweiten Teststelle 7 ein zweiter Rezeptor 8 immobilisiert sind. Der erste Rezeptor 6 ist für einen ersten, in der zu analysierenden Probe enthaltenen Liganden 9 und der zweite Rezeptor 8 für einen zweiten, in der Probe enthaltenen Liganden bindungsspezifisch. Der zweite Ligand weist eine größere Konzentration in der Probe auf als der erste Ligand 9.

An der ersten Teststelle 5 ist direkt unter dem ersten Rezeptor 6 ein erster optischer Sensor 10 und an der zweiten Teststelle 7 direkt unter dem zweiten Rezeptor 8 ein zweiter optischer Sensor 11 in das Halbleiter-Substrat integriert. Zusätzlich ist ein dritter optischer Sensor 12 in die Wandung der Messkammer angeordnet, der nicht mit einem Rezeptor bedeckt ist und als Referenzwertgeber dient. Die Sensoren 10, 11, 12 können beispielsweise Photodioden sein.

Die Vorrichtung 1 weist ferner eine Mischeinrichtung 1 3 auf, welche die Einlassöffnung 3 der Messkammer 2 mit einer Zuführöffnung 14 verbindet. Die Mischeinrichtung 13 dient dazu, die Probe mit einem für den zweiten Rezeptor 8 bindungsspezifischen Kompetitor 15 zu vermischen, der mit einem Enzym 16, wie z.B. HRP (Meerrettich-Peroxidase), markiert ist. Dazu wird die Probe beispielsweise mit Hilfe einer Pipette oder einer Pumpe durch die Zuführöffnung 14 hindurch in einen Aufnahmeraum 16 der Mischeinrichtung 13 eingeleitet. In dem Aufnahmeraum 17 ist der markierte Kompetitor 15 in stabilisierter Form derart relativ zu der Zuführöffnung 14 angeordnet, dass die Probe während und/oder nach dem Einleiten in den Aufnahmeraum 17 mit dem Kompetitor 15 bzw. dem damit verbundenen Enzym 16 in Kontakt gerät und sich mit den Kompetitor-Enzym-Komplexen vermischt. In Strömungsrichtung hinter dem Aufnahmeraum 17 weist die Mischeinrichtung 1 3 eine als Möbiusmischer ausgestaltete Mischerstruktur 18 auf, die mit ihrem einen Ende mit dem Aufnahmeraum 17 und mit ihrem anderen, eine Abgabeöffnung für das aus der Probe und den Kompetitor-Enzym-Komplexen gebildete Gemisch aufweisenden Ende über einen Kanal 19 mit der Einlassöffnung 3 der Messkammer 2 verbunden ist.

Die Mischeinrichtung 13 ist derart ausgestaltet, dass der Kompetitor 15 in der Mischkammer 2 in einer vorgegebenen, einem zu messenden Grenzwert entsprechenden Konzentration in dem Gemisch vorliegt. Dies wird dadurch erreicht, dass die Messkammer 2 und die Mischeinrichtung 13 ein vorbestimmtes Volumen aufweisen, und dass die Menge des in dem Aufnahmeraum 17 stabilisierten Kompetitors 15 so gewählt ist, dass sich die gewünschte Konzentration einstellt, wenn der Kompetitor 15 mit der Probe zu einem Gemisch vermischt wird, welches das vorbestimmte Volumen aufweist.

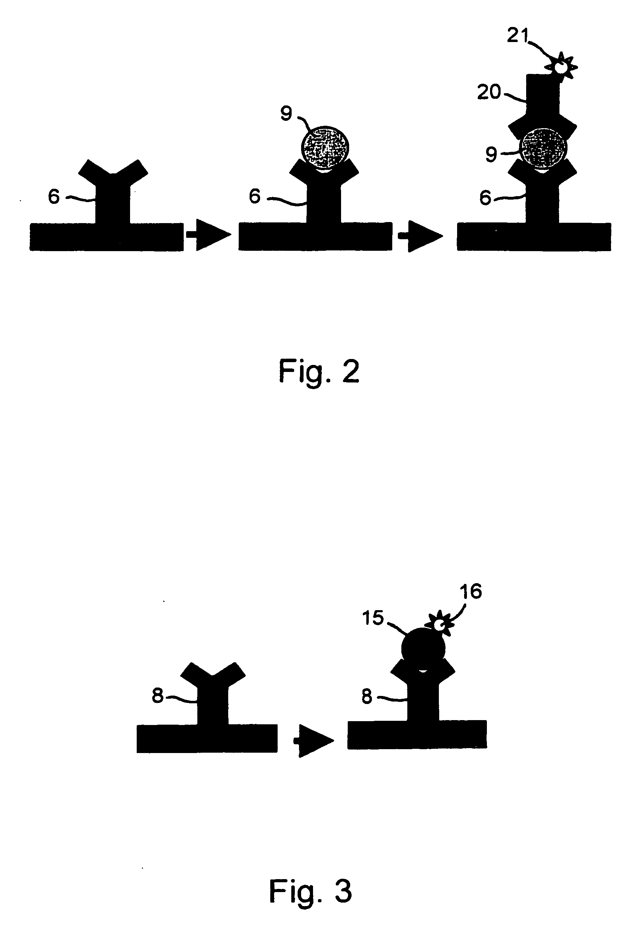

Nachdem die Messkammer 2 mit dem aus der Probe und den Kompetitor-Enzym-Komplexen gebildeten Gemisch befüllt wurde, wird eine vorgegebene erste Zeitdauer abgewartet, um den in dem Gemisch enthaltenen Liganden und dem Kompetitor 15 die Möglichkeit zu geben, jeweils an die für sie bindungsspezifischen Rezeptoren 6, 8 zu binden. Die erste Zeitdauer wird so gewählt, dass jeweils nur ein Teil der an den einzelnen Teststellen 5, 7 vorhanden freien Bindungsstellen der Rezeptoren 6, 8 an einen Liganden bindet. In Fig. 2 ist das Binden eines Moleküls des ersten Liganden 9 an den ersten Rezeptor 6 und in Fig. 3 das Binden eines Kompetitor-Enzym-Komplexes an den zweiten Rezeptor 8 schematisch dargestellt.

Nachdem die vorgegebene erste Zeitdauer abgelaufen ist, wird über die Zuführöffnung 14, die Einlassöffnung 3 und die Auslassöffnung 4 eine Spülflüssigkeit durch die Messkammer 2 hindurchgeleitet, welche die nicht an einen Rezeptor 6, 8 gebundenen Bestandteile des gebildeten Gemischs aus der Messkammer 2 entfernt.

Danach wird eine Lösung in die Messkammer 2 eingebracht, die einen Nachweisantikörper 20 enthält, der für den ersten Liganden 9 bindungsspezifisch ist und mit einem Enzym 21, wie z.B. HRP, markiert ist. Nachdem der Nachweisantikörper 20 in die Messkammer 2 eingebracht wurde, wird eine vorgegebene zweite Zeitdauer abgewartet, die so gewählt ist, dass nahezu alle an den ersten Rezeptor 6 gebundenen Moleküle des ersten Liganden 9 jeweils an ein Molekül des Nachweisantikörpers 20 binden und dadurch indirekt mit dem Enzym 21 markiert werden.

Nachdem die vorgegebene zweite Zeitdauer abgelaufen ist, wird erneut die Spülflüssigkeit durch die Messkammer 2 hindurchgeleitet, um nicht an einen ersten Liganden gebundene Nachweisantikörper 20 aus der Messkammer 2 zu entfernen.

Danach wird über die Zuführöffnung 14 und die Einlassöffnung 3 ein Chemi-Lumineszenzsubstrat in die Messkammer 2 eingeleitet, das Wasserstoffperoxid und einen Chemiluminophor, wie z.B. Luminol, enthält. Wenn die Enzyme 16, 21 mit dem Wasserstoffperoxid in Kontakt geraten, werden freie Sauerstoffradikale von dem Wasserstoffperoxid abgespalten, wodurch der Chemiluminophor unter Abgabe von Lumineszenzstrahlung chemisch zersetzt wird. Die Lumineszenzstrahlung wird also einerseits in Abhängigkeit von der Bindung des ersten Liganden an den ersten Rezeptor 6 und andererseits in Abhängigkeit von der Bindung des Kompetitors 15 an den zweiten Rezeptor 8 in der Messkammer 2 erzeugt.

Die optischen Sensoren 10, 11 sind für die Lumineszenzstrahlung empfindlich und derart relativ zu den Rezeptoren 6, 8 angeordnet, dass sie jeweils nur die an der ihnen zugeordneten Teststelle 5, 7 erzeugte Lumineszenzstrahlung detektieren, nicht jedoch die Lumineszenzstrahlung, die an der jeweils anderen Teststelle 7, 5 erzeugt wird.

Mit den Sensoren 10, 11 ist eine in der Zeichnung nicht näher dargestellte Auswerteeinrichtung verbunden, die in Abhängigkeit von dem Messsignal des ersten Sensors 10 die Konzentrationen des ersten Liganden 6 in der Probe und in Abhängigkeit von dem Messsignal des zweiten Sensors 11 und der bekannten Konzentration des Kompetitors 15 in dem Gemisch mit Hilfe des Massenwirkungsgesetzes die Konzentration des zweiten Liganden in der Probe bestimmt. Die Auswerteeinrichtung kann als elektrische Schaltung in das Substrat bzw. die Wandung der Messkammer 2 integriert sein.

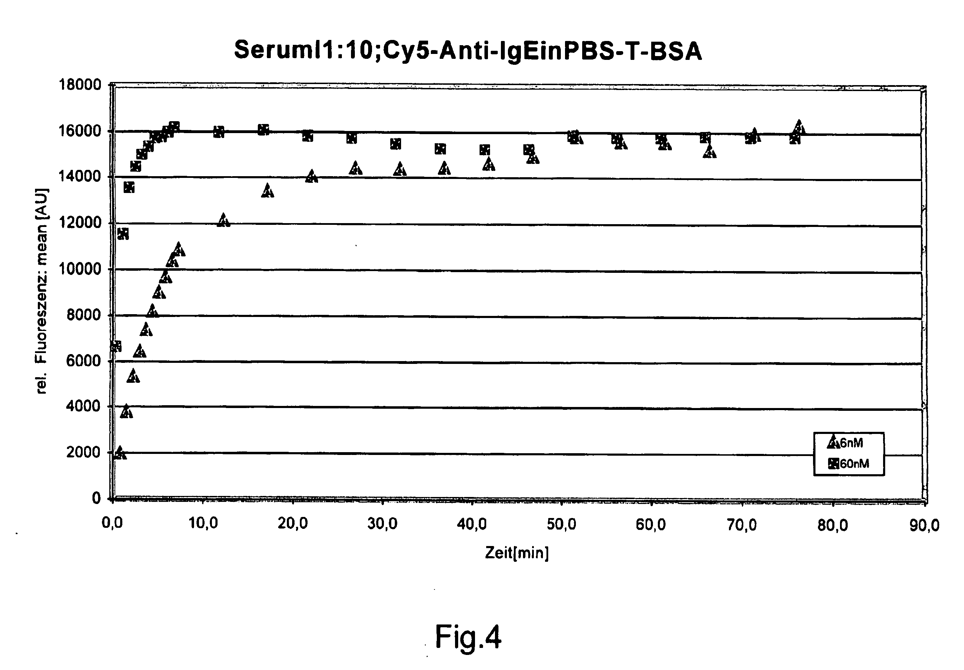

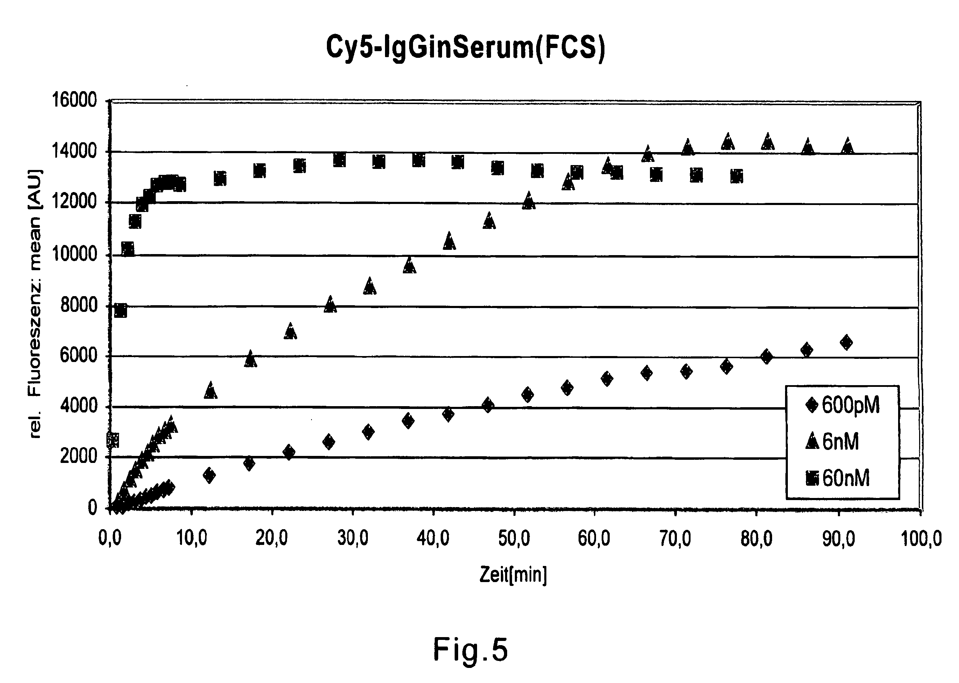

In Fig. 4 und 5 sind mit einem direkten ELISA und einem Sandwich ELISA ermittelte Messwerte als Funktion der Zeit graphisch dargestellt. Bei dem ELISA wird in Abhängigkeit von der Bindung des ersten Liganden an den einen Rezeptor und in Abhängigkeit von der Bindung des zweiten Liganden an einen anderen Rezeptor jeweils eine Lumineszenzstrahlung erzeugt. Die Liganden werden dazu mit Hilfe eines Markers, wie z.B. dem Farbstoff Cy5, markiert und mit einer Anregungsstrahlung bestrahlt, bei welcher der Marker zur Abgabe von Lumineszenzstrahlung angeregt wird. Deutlich ist erkennbar, dass der Anstieg der Messwerte mit abnehmender Konzentration der Liganden in der Probe flacher wird. Durch das erfindungsgemäße Verfahren wird es möglich, die Konzentration zweier in einer Probe in stark unterschiedlicher Konzentration enthaltener Liganden gleichzeitig in der Messkammer 2 zu messen. Dies wird dadurch erreicht, dass die Konzentration des die hohe Konzentration aufweisenden Liganden mit Hilfe eines kompetitiven Assays und die Konzentration des die geringe Konzentration aufweisenden Liganden 9 mit Hilfe eines nicht kompetitiven Assays gemessen werden, so dass bei der Messsignalgewinnung keiner der Sensoren 10, 11 in die Begrenzung gerät.

## Patentansprüche

1. Verfahren zum Bestimmen der Konzentrationen von mindestens zwei Liganden, von denen vermutet wird, dass sie in einer zu analysierenden Probe enthalten sind, wobei ein erster Ligand (9) für einen ersten Rezeptor (6) und ein zweiter Ligand für einen zweiten Rezeptor (8) bindungsspezifisch ist, wobei der erste Rezeptor (6) an mindestens einer ersten Teststelle (5) und der zweite Rezeptor (8) an mindestens einer zweiten Teststelle (7) auf einem Substrat immobilisiert werden, wobei ein für den zweiten Rezeptor (8) bindungsspezifischer Kompetitor (15) mit der Probe vermischt wird, wobei das Gemisch derart mit dem Substrat in Kontakt gebracht wird, dass der erste Ligand (9) an den ersten Rezeptor (6) und der zweite Ligand und/oder der Kompetitor (15) an den zweiten Rezeptor (8) binden können, wobei danach Bestandteile des Gemischs, die nicht an einen immobilisierten Rezeptor (6, 8) gebunden sind, von dem Substrat entfernt werden, wobei ein erstes Messsignal und ein von der Konzentration des an den zweiten Rezeptor (8) gebundenen Kompetitors (15) abhängiges zweites Messsignal erzeugt werden, **dadurch gekennzeichnet, dass** der für den zweiten Rezeptor (8) bindungsspezifische Kompetitor (15) derart mit der Probe vermischt wird, dass der Kompetitor (15) in einer vorgegebenen Konzentration in dem so erhaltenen Gemisch vorliegt, dass das erste Messsignal derart erzeugt wird, dass es von der Konzentration der an den ersten Rezeptor (6) gebundenen ersten Liganden (9) abhängig ist, und dass mit Hilfe des ersten Messsignals die Konzentration des ersten Liganden (9) in der Probe und mit Hilfe des zweiten Messsignals und der bekannten Konzentration des Kompetitors (15) in dem Gemisch die Konzentration des zweiten Liganden in der Probe bestimmt werden.

2. Verfahren nach Anspruch 1, **dadurch gekennzeichnet, dass** ein für den ersten Liganden (9) bindungsspezifischer, mit einem ersten Marker markierter Nachweisantikörper (20) mit der ersten Teststelle (5) in Kontakt gebracht wird, dass danach nicht an einen immobilisierten Rezeptor (56, 8) gebundene Marker von dem Substrat entfernt werden, und dass danach das erste Messsignal in Abhängigkeit von der Konzentration des ersten Markers erzeugt wird.

3. Verfahren nach Anspruch 1 oder 2, **dadurch gekennzeichnet, dass** der Kompetitor (15) mit einem zweiten Marker markiert wird, und dass nach dem Entfernen der nicht an einen immobilisierten Rezeptor (6, 8) gebunden Bestandteile des Gemischs von dem Substrat das zweite Messsignal in Abhängigkeit von der Konzentration des an den zweiten Rezeptor (8) gebundenen zweiten Markers erzeugt wird.

4. Verfahren nach einem der Ansprüche 1 bis 3, **dadurch gekennzeichnet, dass** der erste Marker und/oder zweite Marker ein Enzym (16, 21) ist (sind), dass das Enzym (16, 21) während der Erfassung der Messsignale mit mindestens zwei Chemikalien in Kontakt gebracht wird, zwischen denen bei Anwesenheit des Enzyms eine chemische Redoxreaktion auftritt, und dass das erste Messsignal und/oder das zweite Messsignal durch Messen eines Redoxpotentials erzeugt wird (werden).

5. Verfahren nach einem der Ansprüche 1 bis 4, **dadurch gekennzeichnet, dass** der erste Marker und/oder zweite Marker während der Erfassung der Messsignale mit einer Anregungsstrahlung bestrahlt wird (werden), welche den (die) Marker zur Abgabe einer Lumineszenzstrahlung anregt, und dass das erste Messsignal und/oder das zweite Messsignal durch Messen der Lumineszenzstrahlung des betreffenden Markers erzeugt wird (werden).

6. Verfahren nach einem der Ansprüche 1 bis 5, **dadurch gekennzeichnet, dass** die erste Teststelle (5) und/oder zweite Testelle (7) während der Erfassung der Messsignale mit einem Chemi-Lumineszenzsubstrat in Kontakt gebracht wird (werden), in dem in Abhängigkeit von der Bindung des ersten Liganden (9) an den ersten Rezeptor (6) und/oder in Abhängigkeit von der Bindung des Kompetitors (15) an den zweiten Rezeptor (8) zur Abgabe einer Lumineszenzstrahlung angeregt wird, und dass das erste Messsignal und/oder das zweite Messsignal durch Messen der Lumineszenzstrahlung an der betreffenden Teststelle (5, 7) erzeugt wird (werden).

7. Vorrichtung (1) zum Bestimmen der Konzentrationen von mindestens zwei Liganden in einer zu analysierenden Probe, mit einer zumindest eine Einlassöffnung (3) und eine Auslossöffnung (4) aufweisenden Messkammer (2), die ein Substrat hat, auf dem an einer ersten Teststelle (5) ein erster Rezeptor (6) und an einer zweiten Teststelle (7) ein zweiter Rezeptor (8) immobilisiert sind, wobei der erste Rezeptor (6) für einen ersten Liganden (9) und der zweite Rezeptor (8) für einen zweiten Liganden bindungsspezifisch ist, wobei der ersten Teststelle (5) ein erster Sensor (10) zum Erfassen eines von der Konzentration des an den ersten Rezeptor (6) gebundenen Liganden abhängiges ersten Messsignal und der zweiten Teststelle (7) ein zweiter Sensor (11) zugeordnet sind, **dadurch gekennzeichnet, dass** die Vorrichtung (1) eine Mischeinrichtung (1 3) aufweist, die zum Vermischen der Probe mit einem für den zweiten Rezeptor (8) bindungsspezifischen Kompetitor (15) mit einer Zuführöffnung (14) für die Probe und einem den Kompetitor (15) enthaltenden Aufnahmeraum (17) verbunden ist, dass die Mischeinrichtung (1 3) eine Abgabeöffnung für das aus der Probe und dem Kompetitor (15) gebildete Gemisch aufweist, die mit der Einlassöffnung (3) der Messkammer (2) verbunden ist, dass die Mischeinrichtung (13) derart ausgestaltet ist, dass der Kompetitor (15) in einer vorgegebenen Konzentration in dem Gemisch vorliegt, dass der zweite Sensor (11) zum Erfassen eines von der Konzentration des an den zweiten Rezeptor (8) gebundenen Kompetitors (15) abhängigen zweiten Messsignals ausgebildet und mit einer Auswerteeinrichtung verbunden ist, die zum Bestimmen der Konzentration des zweiten Liganden in der Probe aus dem zweiten Messsignal und der Konzentration des Kompetitors (15) in dem Gemisch ausgebildet ist.

8. Vorrichtung (1) nach Anspruch 7, **dadurch gekennzeichnet, dass** der Kompetitor (15) in gelförmiger, teigiger oder fester Form in dem Aufnahmeraum (17) stabilisiert ist, vorzugsweise derart, dass er an einer Begrenzungswand des Aufnahmeraums (17) anhaftet, und dass der Aufnahmeraum (17) zum Lösen des Kompetitors (15) in der Probe als Durchflussmischkammer ausgebildet ist, über welche die Zuführöffnung (14) für die Probe mit der Einlassöffnung (3) der Messkammer (2) verbunden ist.

9. Vorrichtung (1) nach Anspruch 7 oder 8, **dadurch gekennzeichnet, dass** die Durchflussmischkammer eine Mischerstruktur (18) aufweist, die derart ausgestaltet ist, dass das Gemisch beim Durchfluss durch die Durchflussmischkammer vorzugsweise in abwechselnd zueinander entgegengesetzt verlaufende Richtungen abgelenkt wird, und dass die Mischerstruktur (18) zwischen dem in gelförmiger, teigiger oder fester Form vorliegenden Kompetitor (15) und der Einlassöffnung (3) der Messkammer (2) angeordnet ist.

10. Vorrichtung (1) nach einem der Ansprüche 7 bis 9, **dadurch gekennzeichnet, dass** die Sensoren (10, 11) optische Sensoren sind, und dass der erste Rezeptor (6) zur Detektion einer in Abhängigkeit von der Bindung des ersten Liganden (9) an den ersten Rezeptor (6) erzeugten ersten Lumineszenzstrahlung vorzugsweise direkt auf dem ersten Sensor (10) und/oder der zweite Rezeptor (8) zur Detektion einer in Abhängigkeit von der Bindung des Kompetitors (15) an den zweiten Rezeptor (8) erzeugten zweiten Lumineszenzstrahlung vorzugsweise direkt auf dem zweiten Sensor (11) angeordnet ist (sind).

11. Vorrichtung (1) nach einem der Ansprüche 7 bis 10, **dadurch gekennzeichnet, dass** der Kompetitor (15) mit einem Marker markiert ist, der durch Bestrahlung mit einer Anregungsstrahlung zur Abgabe der Lumineszenzstrahlung anregbar ist, dass die Vorrichtung (1) zum Bestrahlen der zweiten Teststelle (7) mit der Anregungsstrahlung mindestens eine Strahlungsquelle aufweist, und dass der zweite Sensor (11) für die Lumineszenzstrahlung empfindlich und für die Anregungsstrahlung unempfindlich ist.

12. Kit zur Durchführung des Verfahrens nach einem der Ansprüche 1 bis 6, umfassend
- eine Vorrichtung (1) nach einem der Ansprüche 7 bis 11, bei welcher der erste Sensor (10) ein Sensor zur Messung eines Redoxpotentials ausgebildet ist,
- einen für den ersten Liganden (9) bindungsspezifischen, mit einem Enzym (21) markierten Nachweisantikörper (20) und
- mindestens zwei Chemikalien, zwischen denen bei einem Kontakt mit dem Enzym eine chemische Redoxreaktion auftritt.

13. Kit zur Durchführung des Verfahrens nach einem der Ansprüche 1 bis 6, insbesondere nach Anspruch 12, umfassend
- eine Vorrichtung (1) nach einem der Ansprüche 7 bis 11,
- einen für den ersten Liganden (9) bindungsspezifischen, mit einem Enzym (21) markierten Nachweisantikörper (20) und
- ein Chemi-Lumineszenzsubstrat, in dem bei einem Kontakt mit dem Enzym (21) eine chemische Reaktion ausgelöst wird, bei der eine Lumineszenzstrahlung freigesetzt wird, für die der erste Sensor (10) empfindlich ist.

14. Kit zur Durchführung des Verfahrens nach einem der Ansprüche 1 bis 6, insbesondere nach Anspruch 12 oder 13, umfassend
- einen für den ersten Liganden (9) bindungsspezifischen Nachweisantikörper (20), der mit einem Marker markiert ist, der durch Bestrahlen mit einer Anregungsstrahlung zur Abgabe einer Lumineszenzstrahlung anregbar ist,
- eine Vorrichtung (1) nach einem der Ansprüche 7 bis 11, bei welcher der erste Sensor (10) für die Lumineszenzstrahlung empfindlich und für die Anregungsstrahlung unempfindlich ist, und
- eine Strahlungsquelle, die zur Aussendung der Lumineszenzstrahlung auf die erste Teststelle (5) angeordnet ist.

15. Kit zur Durchführung des Verfahrens nach einem der Ansprüche 1 bis 6, insbesondere nach einem der Ansprüche 12 bis 14, umfassend
- eine Vorrichtung (1) nach einem der Ansprüche 7 bis 11, bei welcher der Kompetitor (15) mit einem Enzym (16) markiert ist und bei welcher der zweite Sensor (11) für zur Messung eines elektrischen Redoxpotentials ausgebildet ist, und
- mindestens zwei Chemikalien, zwischen denen bei einem Kontakt mit dem Enzym (16) eine chemische Redoxreaktion auftritt.

16. Kit zur Durchführung des Verfahrens nach einem der Ansprüche 1 bis 6, insbesondere nach einem der Ansprüche 12 bis 15, umfassend
- eine Vorrichtung (1) nach einem der Ansprüche 7 bis 11, bei welcher der Kompetitor (15) mit einem Enzym (16) markiert ist, und
- ein Chemi-Lumineszenzsubstrat, in dem bei einem Kontakt mit dem Enzym (16) eine chemische Reaktion ausgelöst wird, bei der eine Lumineszenzstrahlung freigesetzt wird, für die der zweite Sensor (11) empfindlich ist.

## Claims

1. Method for determining the concentrations of at least two ligands supposed to be present in a sample to be analysed, wherein a first ligand (9) binds specifically to a first receptor (6) and a second ligand binds specifically to a second receptor (8), wherein the first receptor (6) is immobilized to at least one first test site (5) and the second receptor (8) is immobilized to at least one second test site (7) on a substrate, wherein a competitor (15) that binds specifically to the second receptor (8) is mixed with the sample, wherein the mixture is contacted with the substrate in such a way that the first ligand (9) can bind to the first receptor (6) and the second ligand and/or the competitor (15) can bind to the second receptor (8), wherein components of the mixture, which are not bound to an immobilized receptor (6, 8), are subsequently removed from the substrate, wherein a first measurement signal and a second measurement signal which is a function of the concentration of the competitor (15) bound to the second receptor (8) are generated, **characterized in that** the competitor (15) that binds specifically to the second receptor (8) is mixed with the sample in such a way that the competitor (15) is present at a predefined concentration in the mixture thus obtained, that the first measurement signal is generated in such a way that it is a function of the concentration of the first ligand (9) bound to the first receptor (6), and that the concentration of the first ligand (9) in the sample is determined with the aid of the first measurement signal and the concentration of the second ligand in the sample is determined with the aid of the second measurement signal and the known concentration of the competitor (15) in the mixture.

2. Method according to Claim 1, **characterized in that** a detection antibody (20) that binds specifically to the first ligand (9) and has been labelled with a first marker is contacted with the first test site (5), that markers not bound to an immobilized receptor (56, 8) are subsequently removed from the substrate, and that the first measurement signal is then generated as a function of the concentration of the first marker.

3. Method according to Claim 1 or 2, **characterized in that** the competitor (15) is labelled with a second marker, and that, after the components of the mixture that are not bound to an immobilized receptor (6, 8) have been removed from the substrate, the second measurement signal is generated as a function of the concentration of the second marker bound to the second receptor (8).

4. Method according to any of Claims 1 to 3, **characterized in that** the first marker and/or the second marker is (are) an enzyme (16, 21), that, while the measurement signals are recorded, the enzyme (16, 21) is contacted with at least two chemicals which undergo a chemical redox reaction with one another in the presence of the enzyme, and that the first measurement signal and/or the second measurement signal are (is) generated by measuring a redox potential.

5. Method according to any of Claims 1 to 4, **characterized in that**, while the measurement signals are recorded, the first marker and/or the second marker are (is) irradiated with an excitation radiation which causes the marker(s) to emit a luminescent radiation, and that the first measurement signal and/or the second measurement signal are (is) generated by measuring the luminescent radiation of the relevant marker.

6. Method according to any of Claims 1 to 5, **characterized in that**, while the measurement signals are recorded, the first test site (5) and/or the second test site (7) are (is) contacted with a chemiluminescent substrate in which excitation to cause the emission of a luminescent radiation takes place as a function of the first ligand (9) binding to the first receptor (6) and/or as a function of the competitor (15) binding to the second receptor (8), and that the first measurement signal and/or the second measurement signal are (is) generated by measuring the luminescent radiation at the relevant test site (5, 7).

7. Apparatus (1) for determining the concentrations of at least two ligands in a sample to be analyzed, having a measurement chamber (2) which has at least one inlet orifice (3) and one outlet orifice (4) and has a substrate on which a first receptor (6) is immobilized at a first test site (5) and a second receptor (8) is immobilized at a second test site (7), wherein the first receptor (6) binds specifically to a first ligand (9) and the second receptor (8) binds specifically to a second ligand, wherein a first sensor (10) for recording a first measurement signal as a function of the concentration of the ligand bound to the first receptor (6) has been assigned to the first test site (5) and a second sensor (11) has been assigned to the second test site (7), **characterized in that** the apparatus (1) has a mixing device (13) which is connected to a supply orifice (14) for the sample and to a reception space (17) containing a competitor (15) that binds specifically to the second receptor (8), in order to mix the sample with the competitor (15), that the mixing device (13) has a discharge orifice for the mixture of the sample and the competitor (15), which discharge orifice is connected to the inlet orifice (3) of the measurement chamber (2), that the mixing device (13) is designed for the competitor (15) to be present at a predefined concentration in the mixture, that the second sensor (11) is designed for recording a second measurement signal as a function of the concentration of the competitor (15) bound to the second receptor (8) and is connected to an evaluation device designed for determining the concentration of the second ligand in the sample from the second measurement signal and the concentration of the competitor (15) in the mixture.

8. Apparatus (1) according to Claim 7, **characterized in that** the competitor (15) is stabilized in a gel-like, doughy or solid form in the reception space (17), preferably so as to adhere to a boundary wall of the reception space (17), and that the reception space (17) is designed as a mixing flow chamber for dissolving the competitor (15) in the sample, via which chamber the supply orifice (14) for the sample is connected to the inlet orifice (3) of the measurement chamber (2).

9. Apparatus (1) according to Claim 7 or 8, **characterized in that** the mixing flow chamber has a mixing structure (18) which is equipped in such a way that the mixture flowing through the mixing flow chamber is deflected preferably in alternately opposite directions, and that the mixing structure (18) is arranged between the gel-like, doughy or solid competitor (15) and the inlet orifice (3) of the measurement chamber (2).

10. Apparatus (1) according to any of Claims 7 to 9, **characterized in that** the sensors (10, 11) are optical sensors, and that the first receptor (6) for detecting a first luminescent radiation generated as a function of the first ligand (9) binding to the first receptor (6) is arranged preferably directly on the first sensor (10) and/or the second receptor (8) for detecting a second luminescent radiation generated as a function of the competitor (15) binding to the second receptor (8) is arranged preferably directly on the second sensor (11).

11. Apparatus (1) according to any of Claims 7 to 10, **characterized in that** the competitor (15) is labelled with a marker which can be excited by irradiating with an excitation radiation in order to emit the luminescent radiation, that the apparatus (1) has at least one radiation source for irradiating the second test site (7) with the excitation radiation, and that the second sensor (11) is sensitive to the luminescent radiation and insensitive to the excitation radiation.

12. Kit for carrying out the method according to any of Claims 1 to 6, comprising
- an apparatus (1) according to any of Claims 7 to 11, in which the first sensor (10) is a sensor for measuring a redox potential,
- a detection antibody (20) binding specifically to the first ligand (9) and labelled with an enzyme (21), and
- at least two chemicals which undergo a chemical redox reaction with one another when contacting the enzyme.

13. Kit for carrying out the method according to any of Claims 1 to 6, particularly according to Claim 12, comprising
- an apparatus (1) according to any of Claims 7 to 11,
- a detection antibody (20) binding specifically to the first ligand (9) and labelled with an enzyme (21), and
- a chemiluminescent substrate in which, upon contact with the enzyme (21), a chemical reaction is triggered, releasing a luminescent radiation to which the first sensor (10) is sensitive.

14. Kit for carrying out the method according to any of Claims 1 to 6, particularly according to Claim 12 or 13, comprising
- a detection antibody (20) binding specifically to the first ligand (9) and labelled with a marker which can be excited by irradiating with an excitation radiation in order to emit a luminescent radiation,
- an apparatus (1) according to any of Claims 7 to 11, in which the first sensor (10) is sensitive to the luminescent radiation and insensitive to the excitation radiation, and
- a radiation source which is arranged for emitting the luminescent radiation to the first test site (5).

15. Kit for carrying out the method according to any of Claims 1 to 6, particularly according to any of Claims 12 to 14, comprising
- an apparatus (1) according to any of Claims 7 to 11, in which the competitor (15) is labelled with an enzyme (16) and in which the second sensor (11) is designed for measuring an electric redox potential, and
- at least two chemicals which undergo a chemical redox reaction with one another when contacting the enzyme (16).

16. Kit for carrying out the method according to any of Claims 1 to 6, particularly according to any of Claims 12 to 15, comprising
- an apparatus (1) according to any of Claims 7 to 11, in which the competitor (15) is labelled with an enzyme (16), and
- a chemiluminescent substrate in which, upon contact with the enzyme (16), a chemical reaction is triggered, releasing a luminescent radiation to which the second sensor (11) is sensitive.

## Revendications

1. Procédé pour déterminer les concentrations d'au moins deux ligands, dont on suppose qu'ils sont contenus dans un échantillon à analyser, un premier ligand (9) ayant une capacité de liaison spécifique pour un premier récepteur (6) et un deuxième ligand une capacité de liaison spécifique pour un deuxième récepteur (8), dans lequel on immobilise le premier récepteur (6) sur au moins un premier point d'essai (5) et le deuxième récepteur (8) sur au moins un deuxième point d'essai (7) sur un substrat, on mélange un compétiteur (15) ayant une capacité de liaison spécifique pour le deuxième récepteur (8) à l'échantillon, le mélange étant mis en contact avec le substrat de manière à pouvoir lier le premier ligand (9) au premier récepteur (6) et le deuxième ligand et/ou le compétiteur (15) au deuxième récepteur (8), on élimine ensuite du substrat les constituants du mélange qui ne sont pas liés à un récepteur immobilisé (6, 8), un premier signal de mesure et un deuxième signal de mesure dépendant de la concentration du compétiteur (15) lié au deuxième récepteur (8) étant générés, **caractérisé en ce que** le compétiteur (15) à capacité de liaison spécifique pour le deuxième récepteur (8) est mélangé à l'échantillon de sorte que le compétiteur (15) soit présent en concentration prédéterminée dans le mélange obtenu de la sorte, **en ce que** le premier signal de mesure est généré de manière à dépendre de la concentration du premier ligand (9) lié au premier récepteur (6) et **en ce que** la concentration du premier ligand (9) dans l'échantillon est déterminée à l'aide du premier signal de mesure et la concentration du deuxième ligand dans l'échantillon, à l'aide du deuxième signal de mesure et de la concentration connue du compétiteur (15) dans le mélange.

2. Procédé selon la revendication 1, **caractérisé en ce qu'**un anticorps de détection (20) à capacité de liaison spécifique pour le premier ligand (9), marqué par un premier marqueur, est mis en contact avec le premier point d'essai (5), **en ce que** des marqueurs non liés à un récepteur immobilisé (56, 8) sont ensuite éliminés du substrat et **en ce que** le premier signal de mesure est ensuite généré en fonction de la concentration du premier marqueur.

3. Procédé selon la revendication 1 ou 2, **caractérisé en ce que** le compétiteur (15) est marqué par un deuxième marqueur et **en ce que**, après avoir éliminé du substrat les constituants du mélange non liés à un récepteur immobilisé (6, 8), le deuxième signal de mesure est généré en fonction de la concentration du deuxième marqueur lié au deuxième récepteur (8).

4. Procédé selon l'une quelconque des revendications 1 à 3, **caractérisé en ce que** le premier marqueur et/ou le deuxième -marqueur est/sont une enzyme (16, 21), **en ce que**, pendant l'enregistrement des signaux de mesure, l'enzyme (16, 21) est mise en contact avec au moins deux produits chimiques, entre lesquels se produit une réaction chimique de type redox en présence de l'enzyme, et **en ce que** le premier signal de mesure et/ou le deuxième signal de mesure est/sont généré(s) par mesure d'un potentiel redox.

5. Procédé selon l'une quelconque des revendications 1 à 4, **caractérisé en ce que**, pendant l'enregistrement des signaux de mesure, on expose le premier marqueur et/ou le deuxième marqueur à un rayonnement d'excitation, qui stimule le(s) marqueur(s) afin d'induire l'émission d'un rayonnement luminescent, et **en ce que** le premier signal de mesure et/ou le deuxième signal de mesure est/sont généré(s) par la mesure du rayonnement luminescent du marqueur concerné.

6. Procédé selon l'une quelconque des revendications 1 à 5, **caractérisé en ce que**, pendant l'enregistrement des signaux de mesure, on met en contact le premier point d'essai (5) et/ou le deuxième point d'essai (7) avec un substrat à chimioluminescence, lequel est stimulé pour induire l'émission d'un rayonnement à chimioluminescence en fonction de la liaison du premier ligand (9) au premier récepteur (6) et/ou en fonction de la liaison du compétiteur (15) au deuxième récepteur (8) et **en ce que** le premier signal de mesure et/ou le deuxième signal de mesure est/sont généré(s) par la mesure du rayonnement à chimioluminescence au point d'essai concerné (5, 7).

7. Dispositif (1) pour déterminer les concentrations d'au moins deux ligands dans un échantillon à analyser, avec une chambre de mesure (2) présentant au moins un orifice d'entrée (3) et un orifice de sortie (4), qui a un substrat, sur lequel sont immobilisés un premier récepteur (6) sur un premier point d'essai (5) et un deuxième récepteur (8) sur un deuxième point d'essai (7), dans lequel le premier récepteur (6) a une capacité de liaison spécifique pour un premier ligand (9) et le deuxième récepteur (8) a une capacité de liaison spécifique pour un deuxième ligand, dans lequel on affecte un premier capteur (10) au premier point d'essai (5) pour enregistrer un premier signal de mesure dépendant de la concentration du ligand lié au premier récepteur (6) et un deuxième capteur (11) au deuxième point d'essai (7), **caractérisé en ce que** le dispositif (1) présente un dispositif de mélange (13) qui est relié, pour le mélange de l'échantillon avec un compétiteur (15) à capacité de liaison spécifique pour le deuxième récepteur (8), à un orifice d'alimentation (14) pour l'échantillon et à une chambre de réception (17) contenant le compétiteur (15), **en ce que** le dispositif de mélange (13) présente un orifice d'évacuation pour le mélange formé à partir de l'échantillon et du compétiteur (15), qui est relié à l'orifice d'entrée (3) de la chambre de mesure (2), **en ce que** le dispositif de mélange (13) est conçu de sorte que le compétiteur (15) soit présent dans le mélange en concentration prédéterminée, **en ce que** le deuxième capteur (11) est conçu pour enregistrer un deuxième signal de mesure dépendant de la concentration du compétiteur (15) lié au deuxième récepteur (8) et relié à un dispositif d'évaluation, qui est conçu pour déterminer la concentration du deuxième ligand dans l'échantillon à partir du deuxième signal de mesure et de la concentration du compétiteur (15) dans le mélange.

8. Dispositif (1) selon la revendication 7, **caractérisé en ce que** le compétiteur (15) est stabilisé dans la chambre de réception (17) sous une forme de gel, de pâte ou sous une forme solide, de préférence de manière à adhérer à une paroi de délimitation de la chambre de réception (17), et **en ce que** la chambre de réception (17) se présente sous la forme d'une chambre de mélange de débit pour dissoudre le compétiteur (15) dans l'échantillon, par le biais de laquelle l'orifice de charge (14) pour l'échantillon est relié à l'orifice d'entrée (3) de la chambre de mesure (2).

9. Dispositif (1) selon la revendication 7 ou 8, **caractérisé en ce que** la chambre de mélange de débit présente une structure de mélangeur (18) qui est conçue pour dévier le mélange qui s'écoule à travers la chambre de mélange de débit, de préférence, dans des sens opposés l'une à l'autre en alternance, et **en ce que** la structure du mélangeur (18) est aménagée entre le compétiteur (15) se présentant sous une forme de gel, de pâte ou sous une forme solide et l'orifice d'entrée (3) de la chambre de mesure (2).

10. Dispositif (1) selon l'une quelconque des revendications 7 à 9, **caractérisé en ce que** les capteurs (10, 11) sont des capteurs optiques et **en ce que** l'on agence le premier récepteur (6) permettant de détecter un premier rayonnement luminescent généré en fonction de la liaison du premier ligand (9) sur le premier récepteur (6), de préférence directement sur le premier capteur (10), et/ou le deuxième récepteur (8) permettant de détecter un deuxième rayonnement luminescent généré en fonction de la liaison du compétiteur (15) sur le deuxième récepteur (8), de préférence directement sur le deuxième capteur (11).

11. Dispositif (1) selon l'une quelconque des revendications 7 à 10, **caractérisé en ce que** le compétiteur (15) est marqué par un marqueur, qui peut être excité par un rayonnement d'excitation pour émettre un rayonnement luminescent, **en ce que** le dispositif (1) présente au moins une source de rayonnement pour exposer le deuxième point d'essai (7) au rayonnement d'excitation et **en ce que** le deuxième capteur (11) est sensible au rayonnement luminescent et insensible au rayonnement d'excitation.

12. Trousse pour mettre en oeuvre le procédé selon l'une quelconque des revendications 1 à 6, comprenant :
- un dispositif (1) selon l'une quelconque des revendications 7 à 11, dans lequel le premier capteur (10) se présente sous la forme d'un capteur de mesure d'un potentiel redox,
- un anticorps de détection (20) à capacité de liaison spécifique pour le premier ligand (9), marqué par une enzyme (21), et
- au moins deux produits chimiques, entre lesquels se produit une réaction chimique de type redox lors d'un contact avec l'enzyme.

13. Trousse pour mettre en oeuvre le procédé selon l'une quelconque des revendications 1 à 6, en particulier selon la revendication 12, comprenant :
- un dispositif (1) selon l'une quelconque des revendications 7 à 11,
- un anticorps de détection (20) à capacité de liaison spécifique pour le premier ligand (9), marqué par une enzyme (21), et
- un substrat à chimioluminescence, dans lequel, lors d'un contact avec l'enzyme (21), une réaction chimique est déclenchée, laquelle réaction chimique émet un rayonnement luminescent, auquel est sensible le premier capteur (10).

14. Trousse pour mettre en oeuvre le procédé selon l'une quelconque des revendications 1 à 6, en particulier selon la revendication 12 ou 13, comprenant :
- un anticorps de détection (20) à capacité de liaison spécifique pour le premier ligand (9), qui est marqué par un marqueur, qui peut être excité par exposition à un rayonnement d'excitation pour émettre un rayonnement luminescent,
- un dispositif (1) selon l'une quelconque des revendications 7 à 11, dans lequel le premier capteur (10) est sensible à un rayonnement luminescent et insensible à un rayonnement d'excitation, et
- une source de rayonnement qui est agencée sur le premier point d'essai (5) pour émettre le rayonnement luminescent.

15. Trousse pour mettre en oeuvre le procédé selon l'une quelconque des revendications 1 à 6, en particulier selon l'une quelconque des revendications 12 à 14, comprenant :
- un dispositif (1) selon l'une quelconque des revendications 7 à 11, dans lequel le compétiteur (15) est marqué par une enzyme (16) et dans lequel le deuxième capteur (11) est conçu pour mesurer un potentiel électrique redox, et
- au moins deux produits chimiques, entre lesquels se produit une réaction chimique de type redox lors d'un contact avec l'enzyme (16).

16. Trousse pour mettre en oeuvre le procédé selon l'une quelconque des revendications 1 à 6, en particulier selon l'une quelconque des revendications 12 à 15, comprenant :
- un dispositif (1) selon l'une quelconque des revendications 7 à 11, dans lequel le compétiteur (15) est marqué par une enzyme (16), et
- un substrat à chimioluminescence, dans lequel, lors d'un contact avec l'enzyme (16), une réaction chimique est déclenchée, laquelle réaction chimique émet un rayonnement luminescent, auquel est sensible le deuxième capteur (11).
